(19) Europäisches Patentamt
European Patent Office
Office européen des brevets

(11) Publication number: 0 109 797
A2

(12) EUROPEAN PATENT APPLICATION

(21) Application number: 83306788.7

(22) Date of filing: 08.11.83

(51) Int. Cl.$^3$: H 01 B 3/44
C 08 L 101/00

(30) Priority: 18.11.82 US 442541

(43) Date of publication of application: 30.05.84 Bulletin 84/22

(84) Designated Contracting States: DE FR GB

(71) Applicant: DOW CORNING CORPORATION
3901 S. Saginaw Road
Midland Michigan 48640(US)

(72) Inventor: Vincent, Gary Allen
3121 Bullock Creek Road
Midland Michigan 48640(US)

(74) Representative: Laredo, Jack Joseph et al,
Elkington and Fife High Holborn House 52/54 High Holborn
London, WC1V 6SH(GB)

(54) **Polymeric compositions resistant to electrical and water treeing.**

(57) A composition comprising a polymeric component and a siloxane, having at least one silicon bonded group having the general formula –OX wherein X is an unsaturated hydrocarbon radical, is disclosed. A method of stabilizing polymers against water and electrical treeing, which comprises adding a siloxane having at least one silicon bonded group having the general formula –OX wherein X is an unsaturated hydrocarbon, is further disclosed.

EP 0 109 797 A2

Croydon Printing Company Ltd.

# POLYMERIC COMPOSITIONS RESISTANT TO ELECTRICAL AND WATER TREEING

This invention relates to a composition comprising a polymeric component and a siloxane having at least one silicon bonded group having the general formula -OX wherein X is an unsaturated hydrocarbon radical. This invention further relates to a method of stabilizing polymers against water and electrical treeing which comprises adding a siloxane having at least one silicon bonded group having the general formula -OX wherein X is an unsaturated hydrocarbon radical.

Polymeric compositions are well-known and are used extensively as insulation materials for wire and cable. As an insulator, it is important the composition have various physical and electrical properties, such as resistance to mechanical cut through, stress crack resistance, and dielectric failure. Recent publications have indicated water tree growth and electrical tree growth in the insulation are particularly important problems since they are associated with, though not necessarily totally responsible for, dielectric failure.

An important application for an insulating material is in high voltage transmission and distribution cable, especially in direct buried underground service. Unfortunately, the efficient use of polymeric compositions in high voltage cables is precluded by the degradation process called "treeing". Treeing is an electrical pre-breakdown process. The name is given to the damage, visually resembling trees, which results in a solid dielectric exposed to electrical stress. Treeing can occur and progress as a result of particle discharges,

without discharges in the presence of moisture, and with impulse, ac, or dc voltages.

It is generally believed two different types of trees exist. Trees which form in the presence of water, and in particular at low voltages, are called water or electrochemical trees. When water is absent, the trees which form are called electrical trees.

Although there are many theories concerning the initiation and growth of trees, there is virtual unanimity in the belief they start at an imperfection in the cable. This imperfection can be a small void or a piece of solid contamination.

Several organic additives have been discovered which are quite effective in retarding the growth of both types of trees. Acetophenone is perhaps one of the best known anti-treeing agents in existence. It is a product of the decomposition of dicumylperoxide which has found wide use as a curing agent to produce crosslinked polyethylene. The initial decreased treeing tendency of crosslinked polyethylene is a direct result of the existence of acetophenone in the former. Unfortunately, the effect is only temporary because the acetophenone diffuses out of the polyethylene with time; and the polymer's resistance to treeing becomes the same as uncrosslinked polyethylene.

The prevention of treeing has also been attempted by preparing super clean resin, the inclusion of fillers, and decreasing or eliminating the cable's exposure to steam during crosslinking.

Silicones have found limited use in the area of treeing. Kato, et al. (U.S. Patent Number 3,956,420) discloses the use of a combination of ferrocene, an 8-substituted quinoline, and a silicone liquid to increase

the dielectric strength of polyethylene and its voltage endurance in water. Ashcraft, et al. (U.S. Patent Number 4,144,202) inhibits water treeing in ethylene polymer compositions by employing organosilanes containing an epoxy radical. Ashcraft, et al. (U.S. Patent Number 4,263,158) further discloses the use of organosilanes containing C=N bonds to inhibit water treeing in ethylene polymers. Ashcraft et al. (Canadian Patent Number 12,007) further discloses the use of organosilanes containing C=O bonds to inhibit water treeing in ethylene polymers.

German Offenlegungsschrift Number 2,737,430 and U.S. Patent Number 4,299,713 discloses the addition of alkoxysilanes to polyolefin insulation to prevent water tree formation. U.S. Patent Number 4,332,957 discloses the use of phenoxyalkoxy-substituted silanes as water tree and electrical tree retardant additives. U.S. Patent Number 3,553,348, British Patent Number 1,248,256, and British Patent Number 1,277,378 disclose treating mineral fillers with organosilanes and then adding them to the polymer to decrease the porosity of the composition. Japanese Patent Number Sho 50[1981]-92946 discloses the use of silicon grafted polyolefins in combination with propionates to inhibit water treeing. Japanese Patent Number Sho 56[1981]-109494 discloses the use of diorganopolysiloxanes having a viscosity range of 30 to 500 centistokes to inhibit water treeing. This patent further discloses siloxanes modified with alkoxy groups have little effect upon water treeing.

As is evidenced by the prior art, treeing can be inhibited in two different ways. If the voids in the plastic are filled, there is slight improvement in resistance to treeing. If voltage stabilizers, such as acetophenone, are included in the polyethylene, the

stabilizers are thought to trap and deactivate electrons, and thus inhibit treeing. Most, if not all, of the voltage stabilizers are mobile aromatic compounds. The mobility of the compound, however, can not be so great that it does not stay in the plastic. If it migrates to the surface, it evaporates, and its effectiveness is totally lost.

It is theorized that the ideal composition should contain an additive which is mobile and sufficiently compatible (soluble) with the plastic so it can migrate to the voids and solid impurities which are the points of treeing initiation. By filling and surrounding these faults in the plastic, it retards the initiation of the trees; and by filling the tree channel as it is formed, it retards the growth of the trees. At the same time, the additive must be sufficiently nonvolatile to assure it stays in the plastic and does not evaporate.

It is thus an object of this invention to provide a composition comprising a polymeric component and a siloxane having at least one silicon-bonded group having the general formula -OX wherein X is an unsaturated hydrocarbon radical, the siloxane being mobile, nonvolatile, and compatible with the plastic. It is a further object of this invention to provide a method of stabilizing polymers against water and electrical treeing by adding a siloxane having at least one silicon-bonded group having the general formula -OX wherein X is an unsaturated hydrocarbon.

In general, the polymeric component of the present invention can be any solid synthetic organic polymeric thermoplastic resin. Specific examples of

suitable resins include polyolefins and copolymers thereof, vinyls, olefin-vinyl copolymers, olefin-allyl copolymers, polyamides, acrylics, polystyrenes, cellulosics, polyesters, and fluorocarbons.

The polyolefins include solid polymers of olefins, particularly mono-alpha-olefins, which comprise from about two to about six carbon atoms, e.g., polyethylene, polypropylene, polybutene, polyisobutylene, poly(4-methyl pentane), and the like. Copolymers of ethylene, and other compounds interpolymerizable with ethylene such as butene-1, pentene-1, styrene, and the like, may be employed. In general, the copolymer will be comprised of 50 percent by weight or more of ethylene.

Suitable examples of vinyl polymers include polyvinyl chloride, polyvinyl acetate, vinyl chloride/vinyl acetate copolymers, polyvinyl alcohol, and polyvinyl acetal.

Suitable examples of olefin-vinyl copolymers include ethylene-vinyl acetate, ethylene-vinyl propionate, ethylene-vinyl isobutyrate, ethylene-vinyl alcohol, ethylene-methyl acrylate, ethylene-ethyl acrylate, ethylene-ethyl methacrylate, and the like. In general, the ethylene constitutes at least 25 percent by weight of the copolymer.

Specific examples of suitable olefin-allyl copolymers include ethylene-allyl benzene, ethylene-allyl ether, and ethylene-acrolein.

It is preferred, however, that the polymer be a polyolefin, with polyethylene being most preferred.

As far as is known at this time, the siloxane of the present invention can be any siloxane as long as it contains at least one silicon-bonded group having the general formula -OX wherein X is an unsaturated

hydrocarbon radical. It is believed that the unsaturation is necessary for mobility of the additive in the polymer. Examples of suitable unsaturated hydrocarbon radicals include alkenyl, cycloalkenyl, alkynyl, aryl, alkaryl, aralkyl, alkenylaryl, alkynylaryl, and corresponding substituted unsaturated hydrocarbon radicals. Specific examples of suitable unsaturated hydrocarbon radicals include alkenyl radicals such as the vinyl, allyl, butylene, hexylene, octylene, or decylene radicals; cycloalkenyl radicals such as the cyclopentene or cyclohexene radicals; alkynyl radicals such as the acetylene, propyne, butyne, pentyne, or decyne radicals; aryl radicals such as the phenyl, 2-naphthyl, 2-anthracyl, or biphenyl radicals; alkaryl radicals such as the 4-methylphenyl, 2,4-diethylphenyl, or 4-dodecylphenyl radicals; aralkyl radicals such as the benzyl, furfuryl, beta-phenylethyl, or 3,5-dimethylphenylethyl radicals; alkenylaryl radicals such as the phenylacetylene or diphenylacetylene radicals; and the corresponding substituted unsaturated hydrocarbon radicals such as the dichlorophenyl, nitrophenyl, or trifluorovinyl radicals. It is preferred, however, that the unsaturated hydrocarbon radical, X, be an aromatic radical. It is further preferred that the aromatic radical be selected from the group consisting of the naphthyl, benzyl, and furfuryl radicals. It is optimal that the siloxane be selected from the group consisting of

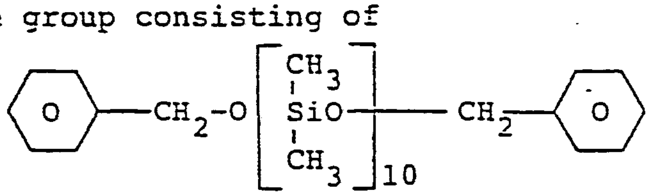 and

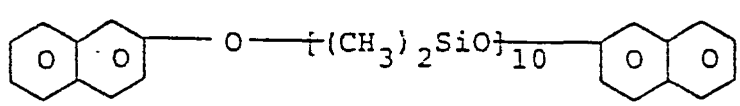 .

Specific examples of suitable siloxanes include those as disclosed in U.S. Patent Number 4,196,131 such as

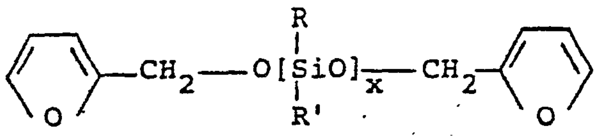

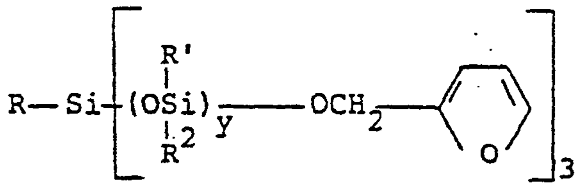

and

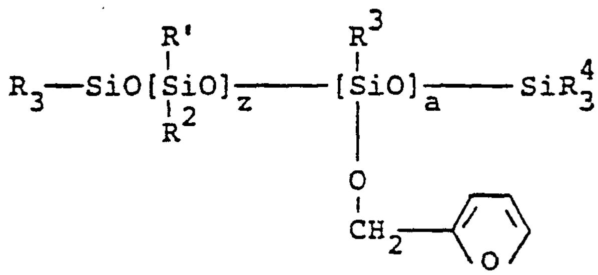

wherein R, R', $R^2$, $R^3$, and $R^4$ are the same or different and selected from the group consisting of monovalent hydrocarbon radicals having from 1 to 10 carbon atoms; x has a value from about 2 to about 100, y has a value of about 1 to about 35, and a and z each have a value of about 1 to about 50.

Further examples of suitable siloxanes include those as disclosed in U.S. Patent Number 4,147,646 such as

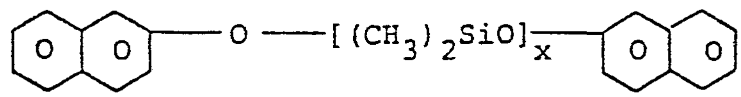 ,

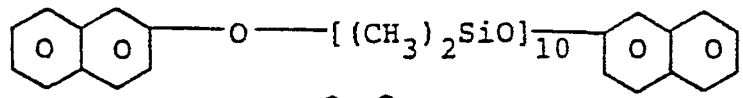 ,

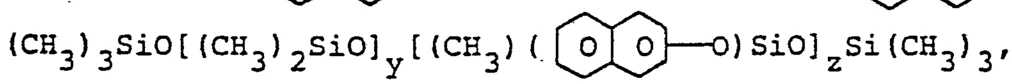

and 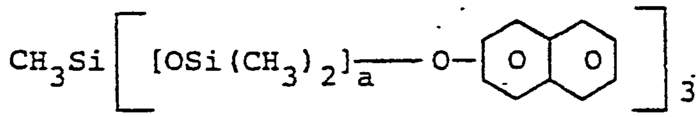

wherein x has an average value ranging from about 2 to about 100, y has an average value ranging from 1 to 10, z has an average value ranging from 1 to 50, the number of y units being at least as great as the number of z units, and a has an average value ranging from about 1 to 35.

Siloxanes disclosed in U.S. Patent Number 4,172,805 have also been found to be useful to control treeing in polyethylene. These siloxanes include

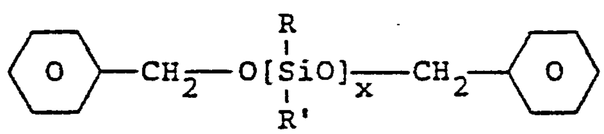 ,

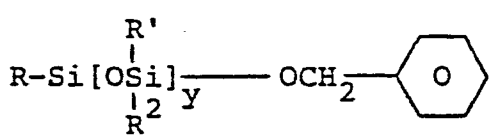 ,

and 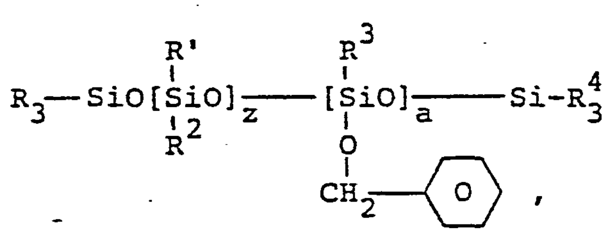 ,

wherein R, R', $R^2$, $R^3$, and $R^4$ are the same or different and selected from the group consisting of monovalent hydrocarbon radicals having from 1 to 10 carbon atoms, x has a value of about 2 to about 100, y has a value of from

about .1 to 35, and a and z each have a value of from about 1 to about 50.

As far as is known at this time, the nature of the other substituents on the siloxane is not critical. It is preferred, however, that they be selected from the group consisting of methyl and phenyl.

As far as is known at this time, the order of mixing the components and the specific procedure employed is not critical for the purpose of this invention. The components may be mixed on a variety of apparatus including multi-roll mills, screw mills, continuous mixers, compounding extruders, and Banbury mixers.

The treeing resistance of the plastic is affected by the amount of additive present and the amount of the -OX moiety in the siloxane. Siloxanes having a higher percentage of substituents having the general formula -OX will have increased treeing resistance. Also, the more additive that is present the more resistant to treeing the plastic is. The amount of additive which would be used would be determined by at least three factors:

1. The level of tree resistance desired- normally this would be as high as possible.
2. The physical properties of the composition - Excessive silicone could result in a composition with insufficient integrity for the application. Excessive silicone could also adversely affect the molding process by causing slippage.

3. The economics of the composition - the more silicone that is used the more expensive the composition.

Based on these factors, it is recommended that the composition contain between one and 10 percent of the siloxane, with 1 to 5 percent preferred.

It is further recommended that the chain length of the siloxane be in the range of 2 to 100.

Minor amounts of other additives may also be employed in conventional amounts to obtain the desired results. Conventional antioxidants such as the hindered phenols, polyquinolines, and the like may be employed. Other ingredients that may be included are plasticizers, dyes, pigments, heat and light stabilizers, antistatic agents, and the like.

The compositions of the invention find particular utility in high voltage transmission and distribution cables but are useful in other electrical applications where a unique combination of enhanced water treeing and electrical treeing properties are needed.

Now in order that those skilled in the art may better understand how the present invention can be practised, the following examples are given by way of illustration and not by way of limitation. All parts and percents are by weight and all viscosities are measured at 25°C unless otherwise specified.

Example

Sample Preparation

Polyethylene beads were surface coated with 5 percent by weight of the siloxane to be evaluated. The wet beads were then fed into a twin screw extruder. The extrudate was cooled in a water bath, blow dried with an air knife, and cut into small beads with a string cutter.

The beads were further mixed to improve the uniformity of the sample. The amount of fluid in the polyethylene was then determined by silicon analysis using atomic absorption. The analysis was performed by hardening via chilling a 10 gram sample of the beads. It was then ground into a powder and 300 mg. was analyzed for silicon. The nonuniformity of the compounding process and the relatively small analytical test sample size led to at least some degree of uncertainty as to the exact amount of silicone present. The siloxanes evaluated were

(A)

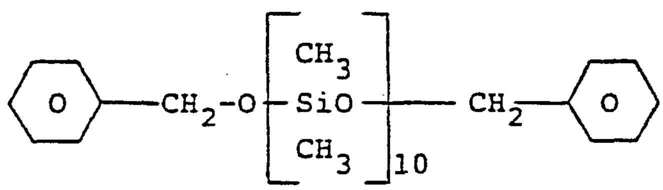

(B)

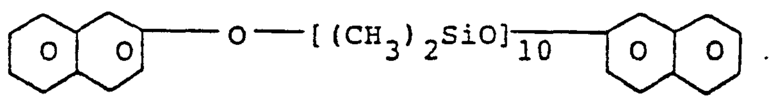

Composition (A) was analyzed to contain .88 percent by weight of Siloxane (A). Composition (B) was analyzed to contain 3.3 percent by weight of Siloxane (B) while Composition (C) was analyzed to contain 5.6 percent by weight of Siloxane (B).

The following comparative compositions were prepared in a similar fashion to the above siloxane containing compositions. Compositions (D) and (E) contained no additive, Compositions (F) and (G) contained 5 percent mineral oil and 95 percent polyethylene, Composition (H) contained 2% acetophenone and 98 percent polyethylene, and Composition (I) was Union Carbide Corporation's 6202 NT product consisting essentially of 2 to 3 percent of a silane additive of the general formula

$C_6H_5C{=}N(CH_2)_3Si(OCH_2CH_3)_3$ and 95 percent polyethylene. The polyethylene used in Compositions (A), (C), (D), and (F) was manufactured by Dow Chemical Company, while the polyethylene used in Compositions (B), (E), (G) and (H) was U.S.I. 31006.

Injection Molding of Samples

A single cavity injection mold was used and the molding temperatures were in the range of 375 to 500°F. The injection pressure ranged from 600 to 900 psi. The mold temperature ranged from 20 to 110°F and the cycle time varied from 45 to 60 seconds.

Electrical Endurance Test

The treated plastic was injected around methylene chloride cleaned electrodes held in place by demountable electrode holders. Samples (B), (E), (G), and (H) were injected around an electrodes which was a steel phonograph needle having a point of 50±5 micrometers radius of curvature while samples (A), (C), (D), and (F) were injected around a precision needles with a 5.0 μm radius of curvature. Specifically, the steel phonograph needle electrode was a Dean #18 Filter Point Needle purchased from John Dean, Inc., Putnam, Connecticut. The precision needles were obtained from the Ogura Jewel Industry Company, Inc., Tokyo, Japan. The other electrode was a standard eighth inch (3.18 mm) steel dowel pin with one end ground to a number four grind micro surface. By adjusting the length of the electrodes protruding from the holders, an electrode spacing of 0.025 inch (0.635mm) was established.

Following the formation of the samples by injection molding, they were x-rayed to permit identification and rejection of those in which the electrodes had moved during molding. Samples having approximately 0.025 ± 0.001 inch (0.635 ± 0.025 mm) gap were selected for

testing. The x-rays were taken by placing the sample in a Plexiglas® holder, positioning it over a Type 51 high contrast 4x5 inch (100x125mm) Polaroid® film and then exposing the sample and film to x-rays. The pictures were then examined under a microscope. The molded sample itself was half an inch (12.5 mm) in diameter and three quarters of an inch (18.75 mm) long.

Samples (B), (E), (G), and (H) were subjected to an electrical voltage of 30,000 volts. Samples (A), (C), (D), and (F) were subjected to an electrical voltage of 8,000 volts. The samples were immersed in oil to prevent flashover. The time to failure was recorded for each sample. Generally, 30 or more samples of each composition were required for statistical evaluation. The data were evaluated by use of the computer program described by G.C. Stone and J.F. Lawless' article entitled "The Application of Weibull Statistics to Insulation Ageing Tests", IEEE Trans. Electr. Insul., EI-14, October, 1979, pp 233-239.

Water Treeing Test

The water treeing tests were done on injection molded samples approximately 1 1/4 inches (31.76 mm) high by 1 inch (25.4 mm) in diameter with an inner cavity approximately 3/4 inch (18.75 mm) in diameter. The bottom of the test sample had an intentional fault with a radius of curvature of approximately 10 micrometers. This point was located 0.025 inch (0.635 mm) from the outside bottom surface of the test sample. Prior to testing, the bottom of the sample was sprayed with conductive silver paint. The test was run in a rig which holds 10 samples. Each sample was charged with 2 ml of saturated aqueous sodium chloride solution containing a small amount of Triton X-400 (trade mark) surfactant, stearyl benzyl dimethyl ammonium chloride manufactured by Rohm and Haas. A high voltage electrode was inserted in

the salt solution in the test sample. The sample was placed in a small plastic cup containing a ground lead. A voltage of 10kV at 3kHz was applied to the high voltage electrode. Samples were tested in periods ranging from 50 to 400 hours. After the test, the fault was cut out of the test cell with a cork bore and placed immediately in a concentrated aqueous solution of methylene blue containing small amounts of ammonium hydroxide and Triton X-400. The sample was microtomed and the slice containing the point of fault was placed on a glass slide, flooded with the methylene blue solution, and photomicrographed. The length of the trees were measured for an entire sample set. The results of the analysis indicate that the additives do enhance polyethylene's resistance to water treeing. The water tree test performed was similar to the procedure described in U.S. Patent No. 4,144,202.

Claims:

1. A composition comprising a polymeric component and a siloxane having at least one silicon bonded group having the general formula -OX wherein X is an unsaturated hydrocarbon radical.

2. A composition as defined in Claim 1 wherein the unsaturated hydrocarbon radical, X, is an aromatic radical.

3. A composition as defined in Claim 2 wherein the polymeric component is a polyolefin and the aromatic radical is selected from the group consisting of naphthyl, benzyl, and furfuryl radicals.

4. An electrical cable comprising the composition as defined in Claim 1.

5. A method of stabilizing a polymer against water and electrical treeing which comprises adding a siloxane having at least one silicon bonded group having the general formula -OX wherein X is an unsaturated hydrocarbon radical.